# EUROPEAN PATENT APPLICATION

(11) **EP 3 682 802 A1**
(43) Date of publication of application: **22.07.2020**
(21) Application number: 19151758.0
(22) Date of filing: 15.01.2019
(51) Int. Cl.: A61B 6/00, G01R 33/54, A61B 6/03, A61B 6/04, A61B 5/055

(54) **MEDICAL IMAGING OF MULTIPLE PATIENTS**

(71) Applicant: Koninklijke Philips N.V., 5656 AG Eindhoven (NL)
(72) Inventor: HELLE, Michael Günter, 5656 AE Eindhoven (NL); WEISS, Steffen, 5656 AE Eindhoven (NL); JOHNSON, Mark Thomas, 5656 AE Eindhoven (NL); VUPPALA, Sunil Kumar, 5656 AE Eindhoven (NL); VOGTMEIER, Gereon, 5656 AE Eindhoven (NL)
(74) Representative: de Haan, Poul Erik

(57) **Abstract**

Patient throughput in diagnostic imaging is key, and may be increased by simultaneous preparation, scanning, and removal of multiple patients from the same scanner. Accordingly, wide-bore imaging apparatuses, systems, and an associated method, computer program element, and computer readable media are proposed that can exploit the property of accommodating at least two patients simultaneously whilst adapting the image acquisition configuration of the scanner to enable a higher rate of patient imaging.

## Description

### FIELD OF THE INVENTION

The invention relates to an apparatus for acquiring medical image data, and an associated method for acquiring medical image data, a medical imaging system, a computer program element, and a computer readable medium.

### BACKGROUND OF THE INVENTION

Conventionally, medical imaging modalities for diagnostic medical imaging such as MRI, CT, PET and others are designed only to scan one patient at a time. To obtain an increase in the efficiency of a diagnostic medical imaging facility, it might be possible to image more than one patient simultaneously, as discussed in US 6,023,165. The provision of "wide-bore" medical scanners (such as "wide-bore" and "open" MRI scanners) has made such proposed simultaneous multi-patient imaging schemes more practical.

However, there are still significant practical constraints associated with implementing simultaneous multi-patient imaging in"wide-bore" medical scanners.

### SUMMARY OF THE INVENTION

It is therefore an object of the invention to provide an increased patient throughput of a conventional medical imaging scanner.

According to a first aspect, there is provided an apparatus for acquiring first medical image data from a first patient, and second medical image data from a second patient using the same medical scanner. The apparatus comprises:
- an input interface;
- a processor; and
- an output interface.

The processor is configured to generate first control signals to position a first patient in a first imaging location of a medical scanner; and to communicate the first control signals to the medical scanner via the output interface.

The processor is configured to generate second control signals to position a second patient in a second imaging location of the medical scanner; and to communicate the second control signals to the medical scanner via the output interface.

The processor is configured to acquire first medical image data of a first region of interest of the first patient using the medical scanner via the input interface, wherein prior to, or during, the acquisition of the first medical image data the medical scanner is configured using a first image acquisition configuration generated by the processor and transmitted to the medical scanner via the output interface.

The processor is configured to acquire second medical image data of a second region of interest of the second patient using the medical scanner via the input interface wherein prior to, or during, the acquisition of the second medical image data the medical scanner is configured using a second image acquisition configuration of the medical scanner generated by the processor and transmitted to the medical scanner via the output interface.

The output interface is configured to output the first and second medical image data of the first and second patients.

An effect of this is that it is possible to obtain medical images of at least two patients simultaneously, or in an overlapping time relation, and thus to increase the patient throughput of a diagnostic medical imaging facility.

Optionally, the first medical image data of the first region of interest of the first patient is acquired from the medical scanner via the input interface using a first scan protocol that is the same as a second scan protocol used to acquire the second medical image data of the second region of interest of the second patient.

An effect of this is that when using the same scan protocol to acquire images of first and second patients, the scan complexity can be reduced. For example, when performing liver imaging, the same MRI coil covering the abdominal regions of first and second patients, may be specified to increase the efficiency of the scan procedure.

Optionally, the first medical image data of the first region of interest of the first patient is acquired from the medical scanner via the input interface using a third scan protocol that is different to a fourth scan protocol used to acquire the second medical image data of the second region of interest of the second patient.

For example, the third scan protocol could be a liver imaging protocol of the first patient, and the fourth protocol could be a cardiac imaging protocol of the second patient.

An effect of this is that a first patient may be imaged for a different medical condition, or region of the body, compared to a second patient. For example, a first MRI scan coil suitable for imaging the cranium of the first patient is used during the third scan protocol, and a second MRI scan coil suitable for imaging the abdomen of the second patient is used as during the fourth scan protocol. Although separate body regions of the first and second patients are imaged, there is still an improvement in patient throughput because two patients can still simultaneously be loaded into a wide-bore MRI scanner, in the different parts of their bodies are being imaged.

Optionally, the processor is further configured to obtain, via the input interface a first physiological trigger signal of the first patient using a first physiological sensor configured to monitor the first patient, and to acquire the first medical image data at a time related to the first physiological trigger signal, and to obtain, via the input interface , a second physiological trigger signal of the second patient using a second physiological sensor configured to monitor the second patient, and to acquire the second medical image data at a time related to the second physiological trigger signal.

An effect of this is that when imaging at least first and second patients, the medical scanner can acquire image data of portions of the first and/or second patients at an appropriate time point or range based on the portion of anatomy of the respective first and second patients to be acquired. For example, if a first patient is undergoing a cardiac MRI and a second patient is undergoing a liver MRI, the first medical image data of the first patient is acquired using the first patient's cardiac trace to trigger image acquisition, whereas the second medical image data of the second patient is acquired using the second patient's breathing trace data to trigger the acquisition. This enables a medical imaging scanner to be occupied by two patients simultaneously whilst undergoing the same, or different types of medical imaging. Furthermore, patient-customised image acquisition configurations of the medical imaging scanner can be applied. For example, an MRI shim setting can be set into first and second configurations based upon the trigger signal from the first or second patients.

Optionally, the first medical image data is acquired from the medical scanner by the processor simultaneously, or at an overlapping time period, relative to an acquisition time period of the second medical image data.

An effect of this is that a unified imaging volume and/or image acquisition configuration suitable for first and second patients may be provided to enable fast imaging of the same anatomical region of the patients. For example, two medical diagnostic images of the livers of a first and second patient can be obtained simultaneously or in close time relation to each other.

Optionally, the first medical image data is acquired from the medical scanner at a different time period compared to the acquisition time period of the second medical image data.

An effect of this is that a customised imaging volume and/or different image acquisition configurations for each patient is provided, enabling better resolution images to be captured for each patient.

Optionally the acquisition time of the first medical image data from the medical scanner occurs during the positioning of the second patient in the second imaging location of the medical scanner.

An effect of this is that a higher throughput of the medical imaging system can be provided, because the medical imaging system is able to obtain medical imaging data from a subset of the entire volume of the region of interest of the medical scanner, enabling another patient to be moved into or out of position in a further subset of the entire volume of region of interest of the medical scanner during a medical scan.

Optionally, the first and/or second image acquisition configuration generated by the processor and transmitted to the medical scanner via the output interface comprises a first field of view setting configured to obtain first medical image data of the first region of interest located on the first patient, and wherein the second image acquisition configuration generated by the processor and transmitted to the medical scanner via the output interface comprises a second field of view setting configured to obtain second medical image data of the second region of interest located on the second patient.

Accordingly, whilst imaging two patients, the field of view setting is successively adjusted to provide an improved or optimal image quality with respect to the particular imaged region of each patient.

Optionally, the first and/or second image acquisition configurations of the medical scanner generated by the processor and transmitted to the medical scanner via the output interface comprise a field of view setting of the medical scanner that is substantially the same for the acquisition of the first and the second medical image data.

Accordingly, the same or similar anatomical regions of patients may be imaged at least in pairs to increase patient throughput of a medical scanner.

Optionally, the first and second image acquisition configurations of the medical scanner generated by the processor and transmitted to the medical scanner via the output interface further comprise first and second shim settings, and the processor is further configured to configure the medical scanner using the first shim setting prior to acquiring the first medical image data, and to configure the medical scanner using the second shim setting prior to acquiring the second medical image data.

Optionally, the input interface is configured to receive a motion signal from a motion detection system monitoring the medical scanner.

The processor is configured to monitor the second patient during their positioning at the second imaging location of the medical scanner using the motion signal, to predict a collision condition of the second patient with the first patient and/or the medical scanner based on the motion signal, and to halt the positioning of the second patient in the medical scanner, to adjust a positioning trajectory of the second patient based on the motion signal, and/or to change a second image acquisition configuration of the medical scanner based on the motion signal via a signal transmitted to the medical scanner via to output interface.

An effect of this is that mis-positioning of a first patient in relation to a second patient can be avoided and/or corrected. The collision condition can embrace a situation where a second patient strays into the subset of the field of view allocated for a first patient, for example.

Optionally the processor is configured to acquire, via the input interface physical measurement data of the first and the second patients, and wherein the processor is configured to signal to the medical scanner, via the output interface, to automatically adjust the spatial position in the medical scanner, or during positioning of the patients in the scanner, of a first patient support used to support the first patient and/or a second patient support used to support the second patient.

An effect of this is that dimensional (height, width, weight) data of the first and/or second patients known from a patient database can be used to position first and/or second patient supports to more effectively locate the first and/or second patients in the medical scanner.

According to a second aspect, there is provided a method for acquiring first medical image data from a first patient, and second medical image data from a second patient using the same medical scanner. The method comprises:
- positioning a first patient in a first imaging location of a medical scanner;
- positioning a second patient in a second imaging location of the medical scanner;
- acquiring first medical image data of a first region of interest of the first patient using the medical scanner, wherein prior to, or during, the acquisition of the first medical image data the medical scanner is configured using a first image acquisition configuration of the medical scanner;
- acquiring second medical image data of a second region of interest of the second patient using the medical scanner, wherein prior to, or during, the acquisition of the second medical image data the medical scanner is configured using a second image acquisition configuration of the medical scanner; and
- outputting the first and second medical image data of the first and second patients.

Optionally, the first medical image data of the first region of interest of the first patient is acquired using a first scan protocol that is the same as a second scan protocol used to acquire the second medical image data of the second region of interest of the second patient.

Optionally, the first medical image data of the first region of interest of the first patient is acquired using a third scan protocol that is different to a fourth scan protocol used to acquire the second medical image data of the second region of interest of the second patient.

Optionally, the method further comprises:
- obtaining a first physiological trigger signal of the first patient using a first physiological sensor monitoring the first patient, and acquiring the first medical image data at a time related to the first physiological trigger signal; and
- obtaining a second physiological trigger signal of the second patient using a second physiological sensor monitoring the second patient, and acquiring the second medical image data at a time related to the second physiological trigger signal.

According to a third aspect, there is provided:
- a medical scanner configured to receive at least two patients comprising at least first and second movable patient supports; and
- an apparatus according to the first aspect or its optional embodiments.

The first and second patient supports of the medical imaging system are configured to receive first and second control signals from the output interface of the apparatus, and to position a first patient in a first imaging location of a medical scanner, and to position a second patient in a second imaging position of the medical scanner, wherein prior to, or during, the acquisition of first medical image data the medical scanner is configured using a first image configuration of the medical scanner, and wherein prior to, or during, the acquisition of second medical image data the medical scanner is configured using a second image configuration of the medical scanner.

An effect of this is that a medical scanner is provided that is capable of a significantly improved (theoretically, a doubled) patient throughput.

Optionally, the medical imaging system further comprises:
- first and second physiological sensors configured to obtain a first physiological trigger signal from the first patient, and a second physiological trigger signal from the second patient, and to communicate the first and/or second physiological trigger signals to the apparatus.

Optionally, the medical imaging system further comprises:
- a motion detection system configured to monitor motion of the first and/or second patients in the medical scanner, wherein the motion detection system is configured to predict a collision condition of the second patient with the first patient and/or the medical scanner based on a motion signal detected by the motion detection system, and to transmit the motion detection signal to the apparatus.

Optionally, the medical scanner of the medical imaging system is one of an MRI scanner, a CT scanner, a PET scanner, a SPECT scanner, an MRI-CT scanner, a PET-CT scanner, a PET-MRI scanner.

According to a fourth aspect, there is provided a computer program element for controlling an apparatus according to the first aspect, and/or a medical imaging system according to the second aspect, which, when the computer program element is executed by the apparatus and/or the medical imaging system, is adapted to perform the second aspect.

According to a fifth aspect, there is provided a computer readable medium having stored the computer program element the fourth aspect.

Accordingly, it is a basic idea of the invention to enable the scanning of at least a first and a second patient in a medical scanner by varying the image acquisition configuration of the medical scanner to suit the first and the second patients. The image acquisition configuration can, for example, refer to the shim volume and/or position inside an MRI scanner bore, or the field of view of a medical scanner. The image acquisition configuration is also optionally temporarally related to at least physiological signals measured in the first and/or second patients, for example breathing signals or cardiac signals.

Patient throughput in diagnostic imaging is key, and may be increased by simultaneous preparation, scanning, and removal of multiple patients from the same scanner. Accordingly, wide-bore imaging apparatuses, systems, and an associated method, computer program element, and computer readable media are proposed that can exploit the property of accommodating at least two patients simultaneously whilst adapting the image acquisition configuration of the scanner to enable a higher rate of patient imaging.

Although this application is focussed on the use of wide-bore MRI scanners to image at least two patients, it is in principle possible to use a CT scanner or other types of medical scanner to image at least two patients (especially neonates). The application is, therefore, not limited to the simultaneous imaging of a plurality of patients using an MRI scanner, although in the illustrated and discussed embodiments this case will often be presented.

These, and other, aspects of the invention will be apparent from and elucidated with reference to the following figures and the embodiments described hereinafter.

### BRIEF DESCRIPTION OF THE DRAWINGS

The invention will further be exemplified with reference to the following drawings which are provided for illustrative purposes only
Fig. 1 schematically illustrates an apparatus according to the first aspect.
Fig. 2a illustrates an example of medical scanner triggering for two patients based on exhalation signals of the two patients, according to an embodiment.
Fig. 2b schematically illustrates an example of a screen (that could be provided proximate to a medical scanner) showing a visual exhalation synchronisation approach.
Fig. 3 schematically illustrates a general view of a medical imaging system according to the third aspect capable of simultaneously and/or serially scanning at least two patients.
Fig. 4a and 4b schematically illustrate plan views of a simultaneous medical scanning process comprising two patients lying side-by-side.
Fig. 5 schematically illustrates a side view of a simultaneous medical scanning process comprising two patients inside a medical scanner in a stacked configuration.
Fig. 6a and 6b illustrate plan views of a split medical scanner support.
Fig. 7 schematically illustrates one example of a collision avoidance approach in a two-person stacked medical scanner.
Fig. 8 schematically illustrates a method according to the second aspect.

### DETAILED DESCRIPTION OF EMBODIMENTS

In the field of medical imaging, a lot of effort is made to increase patient throughput. New approaches such as compressed sense MRI imaging enable faster scanning. Many advanced imaging methods that provide quantitative information have become mature, and have entered clinical routine to enable important information to be provided for an improved differential diagnosis. However, conventional diagnostic medical imaging (using, for example, a CT scanner, an MRI scanner, a PET scanner, and the like) involves a diagnostic image process on a serial sequence of patients (in other words, one after the other). However, the diameter of the aperture in such medical imaging scanners is increasing owing to improvements in the imaging technology of such scanners. This has opened the opportunity to image at least two patients whilst located next to each other inside the imaging bore of the same medical scanner. The present application primarily discusses MRI imaging, but the concept of scanning two patients simultaneously or in close relation to each other whilst varying the image acquisition configurations is also applicable to, for example, CT scanning, PET scanning, and the like.

MRI imaging is the modality of choice for imaging infants and small children, because it uses non-ionising radiation and provides a superior soft tissue contrast. However, a lot of MRI imaging sequences require the use of cardiac or respiratory triggering (such as during heart scans, or when abdominal organs are being imaged). Therefore, cardiac or respiratory triggering may be used in many different ways in routine clinical imaging, for example, to prospectively determine the starting point of an acquisition, to start the acquisition at a defined period in time, or even during continuous acquisition and use-triggering retrospectively until all data is acquired.

Currently, an image acquisition configuration is applied to an MRI scanner for one patient inside a one-person medical scanner. However, if more than one patient is imaged simultaneously, or in closely in time, in a wide-bore scanner, the use of several image acquisition configurations is implied, with each image acquisition configuration providing acquisition being adjusted to each patient individually. This enables a throughput enhancement associated with imaging at least two patients simultaneously whilst also providing good quality images.

An "image acquisition configuration" is one, or a combination, of configurations of a medical imaging scanner (such as an MRI scanner) defining the imaging of the patient in space and/or time.

For example, the image acquisition configuration is a field of view of an MRI scanner, such that first and second image acquisition configurations provide a first field of view for a first patient and the second field of view for a second patient, where the first and second fields of view cover different positions of a total field-of-view of an MRI scanner. For example, the first field of view may define an imaging volume appropriate for liver imaging of a first patient, and the second field of view could define an imaging volume appropriate for cardiac imaging of a second patient. In between acquiring first medical image data and second medical image data, the field of view is adapted.

For example, the image acquisition configuration concerns varying the size of a "shimming volume" of an MRI scanner. As known to a person skilled in the art, small deviations to the field strength and direction inside an MRI scanner are used to correct for local inhomogeneities in the field. Imaging a further field of view of a second patient is optionally improved by adapting the shimming volume of a second image acquisition configuration, and may be changed in between the imaging of a first and second patient, for example. Optionally, this may include the optimisation of the shim volume for a first and second patient. (Shimming is a process known to a medical imaging professional correcting local magnetic field in homogeneities by adjusting, for example, static gradients in an MRI scanner. This enables a better, more precise image to be made). Furthermore, the field of view may be different for the first and second patients, or may include an organ of interest in both patients at the same time. This may be achieved by switching different RF imaging coils of an MRI scanner in or out of circuit as appropriate. Furthermore, the acquisition time window may be the same, or different, for each patient.

The image acquisition configuration may concern an image triggering method. For example, the image acquisition configuration may be different to, or the same, for imaging of the first or second patients. For example, the first image acquisition configuration applies cardiac triggering, and the second image acquisition configuration applies respiratory triggering.

Optionally, the image acquisition configuration is preset using a first and second image acquisition data setting stored on a server. Accordingly, previous patient records can be used to appropriately set the first and second image acquisition configurations. For example, a first field-of-view can be set according to known dimensions of a first patient.

Accordingly, the term "image acquisition configuration" may be considered to embrace at least the 3D shim volume of an MRI scanner, the 3D field of view of an MRI or other medical imaging scanner, or the acquisition time and/or acquisition trigger of a medical image. When referring to an MRI scanning system the term "image acquisition configuration" may also refer to the coil sensitivity scans (SENSE), B0- , and B 1-field map settings, for example.

Of course, if medical imaging data of two patients is acquired simultaneously, the image reconstruction of the acquired data would be adapted in post-processing to enable separate images of the first and second patients to be obtained.

Optionally, two or more patients may be positioned in the field of view of the scanner for a simultaneously gated scan in the same scanner volume where the image acquisition configuration comprises first and second triggering methods for optimal data acquisition.

Optionally, a double-decker variant of the patient support is provided capable of supporting a first patient in a subset of the field of view vertically above a second patient.

Optionally, a flexible movable wall separating the at least two patients provides a privacy barrier.

Optionally, first and second patient enclosing modules are provided in order to fit one or both patients into the scanner in subsets of the field of view of the scanner in such a way that they do not disturb each other.

Optionally, the apparatus is configured to acquire first medical imaging data of the first patient whilst a second patient is loaded or unloaded on a further patient support. The further patient support may be loaded from the same direction as that of the first patient, or from the axially opposite direction as that used for the first patient.

Optionally, the apparatus is configured to acquire first and second medical imaging data of the first and second patients in parallel using identical image acquisition configurations.

Optionally, the apparatus is configured to monitor a physiological parameter such as heart rate, or breathing rate which are used as image acquisition configurations for triggering image of the first and second patients.

Optionally, the apparatus is configured to acquire shim settings, and/or coil sensitivity scans, and/or B0- and/or B1-maps that have been measured and stored for each patient individually, and to apply such shim settings as image acquisition configurations during the imaging of the first or the second patients respectively.

Optionally, a first field-of-view can be planned for a first patient and a second field-of-view is planned for a second patient. Optionally, the field of view may overlap first and second patients.

Optionally, first and second patients are imaged simultaneously using the same scanning protocol. In the case of mixed protocol, the placement of the first patient is performed so that an overlapping field-of-view is compatible to a scan protocol for the overlapping area. Individual scan areas are applied in the non-overlapping patient volume.

Optionally, the first medical image data is sorted and reconstructed according to image trigger times measured of the first patient, and the second medical image data is sorted and reconstructed according to image trigger times measured of the second patient.

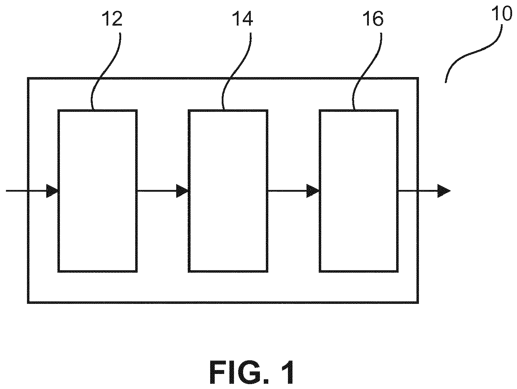

Fig. 1 schematically illustrates an apparatus 10 according to the first aspect. The apparatus 10 is configured to acquire first medical image data from a first patient, and second medical image data from a second patient using the same medical scanner, comprising:
- an input interface 12;
- a processor 14; and
- an output interface 16.

The processor 14 is configured to generate first control signals to position a first patient in a first imaging location of a medical scanner; and to communicate the first control signals to the medical scanner via the output interface 16.

The processor 14 is configured to generate second control signals to position a second patient in a second imaging location of the medical scanner; and to communicate the second control signals to the medical scanner via the output interface 16.

The processor 14 is configured to acquire first medical image data of a first region of interest of the first patient using the medical scanner via the input interface 12, wherein prior to, or during, the acquisition of the first medical image data the medical scanner is configured using a first image acquisition configuration generated by the processor 12 and transmitted to the medical scanner via the output interface 16.

The processor 14 is configured to acquire second medical image data of a second region of interest of the second patient using the medical scanner via the input interface 12, wherein prior to, or during, the acquisition of the second medical image data the medical scanner is configured using a second image acquisition configuration of the medical scanner generated by the processor 14 and transmitted to the medical scanner via the output interface 16. The output interface 16 is configured to output the first and second medical image data of the first and second patients.

The apparatus 10 may be practically invented as a general-purpose processing system such as a personal computer or a high-intensity computer server, or a customised computer having a graphics processing unit GPU. Thus, the input interface 12 is capable of receiving at least imaging data from a medical scanner in a raw, pre-processed, or processed form in addition to optional control signals monitoring patient vital signs such as trigger signals. The input interface 12 may be implemented as at least a DICOM interface, a USB interface, a serial interface, or the like.

The processor 14 may accordingly be a general-purpose computer processor optionally supplemented with a digital signal processor, graphics processing unit, field programmable gate array, and the like.

The output interface 16 may comprise a interface capable of communicating digital medical image data in raw, pre-processed, or fully processed form.

Optionally, the first medical image data of the first region of interest of the first patient is acquired from the medical scanner via the input interface 12 using a first scan protocol that is the same as a second scan protocol used to acquire the second medical image data of the second region of interest of the second patient. In other words, the first and second scan protocols may both be cardiac MRIs, for example.

Optionally, the first medical image data of the first region of interest of the first patient is acquired from the medical scanner via the input interface using a third scan protocol that is different to a fourth scan protocol used to acquire the second medical image data of the second region of interest of the second patient. In other words, the first scan protocol may be a cardiac MRI, and the second scan protocol may be a liver MRI.

A first example is provided of the imaging of an abdominal region (such as the liver) during an expiration phase in MRI. Two patients are positioned inside the scanner bore. The MRI coil is designed in such a way that it covers the abdominal organs of both patients with the aim of imaging the liver in each patient simultaneously. Both patients are equipped with a breathing belt to monitor the physiological motion during breathing.

In this example, patients are not in a condition to hold their breaths, so the scanner settings are chosen to only acquire data during the expiration phase of each patient. A large field of view of the MRI scanner is planned that covers both livers of interest at the same time. The shim settings are optimised accordingly for a simultaneous acquisition of two livers. Accordingly, the shim volume is provided to cover the liver of each patient.

Medical image data is acquired whenever one patient is in the expiration phase. Data is also acquired when both patients are in the expiration phase at the same time.

Only data during the expiration phase is used for image reconstruction. The acquired data is sorted dependent on the expiration phase of each patient. If data was acquired during overlap of both expiration phases, it is used for image reconstruction of both patients.

Optionally, the processor 14 is further configured to:
obtain, via the input interface 12 a first physiological trigger signal of the first patient using a first physiological sensor configured to monitor the first patient, and to acquire the first medical image data at a time related to the first physiological trigger signal; and
obtain, via the input interface 12, a second physiological trigger signal of the second patient using a second physiological sensor configured to monitor the second patient, and to acquire the second medical image data at a time related to the second physiological trigger signal.

In the case of a cardiac triggered approach, the physiological sensor is provided as a heart-rate, echo-Doppler, or pulse monitor, for example. In the case of a breath triggered approach, the physiological sensor is provided as a breathing belt, or a camera observing the patients capable of detecting breathing in patients using post-processing. The skilled person will appreciate that many physiological trigger signals may be obtained using suitable physiological sensors.

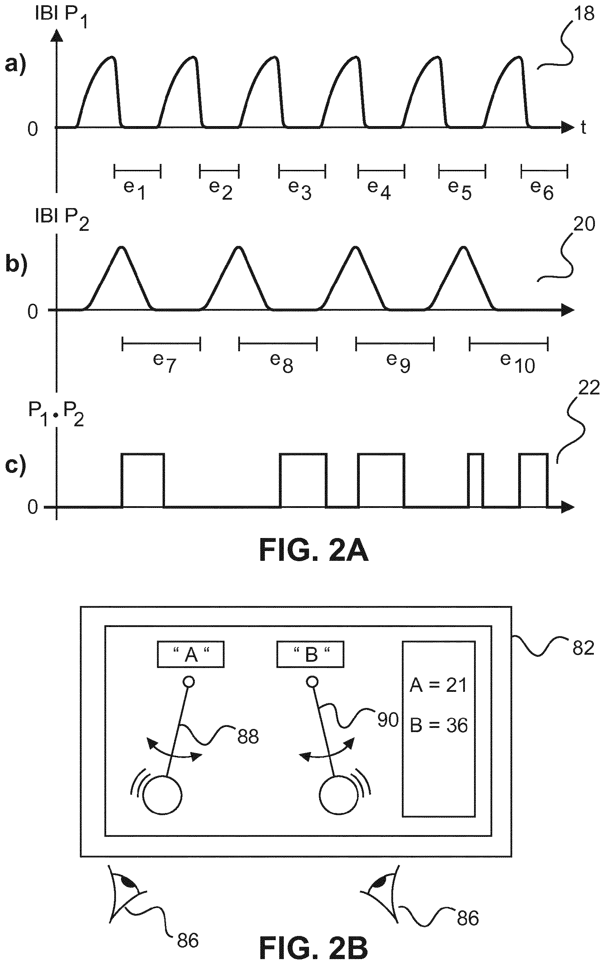

Fig. 2a illustrates waveforms associated with triggering.

Plot 18 illustrates the breathing (chest diameter) of a first patient measured by breathing belt of a first patient P₁ relative to time t. Plot 20 illustrates the breathing (chest diameter) of a second patient P₂ relative to time t. Plot 22 illustrates the logical "AND" of the first and second plots. Ranges e₁ to e₆ illustrate expiration phases of the first patient. Ranges e₇ to e₁₀ illustrate expiration phases of the second patient. Thus, the "logic one" regions of plot 22 denotes time periods when both first and second patients are both in an expiration phase. Accordingly, the medical scanner may be set so as to acquire data of the first and second patients combined in the same field of view on the rising edge of plot 22, for example.

A further example is that of using an MRI scanner for cardiac triggered imaging during fixed acquisition window in the RR-interval.

Initially, two patients are positioned inside the scanner bore. The MRI coil is designed in such a way that it covers the hearts of both patients with the aim to image the chambers of the heart. Both patients are equipped with ECG electrodes to monitor the physiological motion of the heart.

The scanner settings are chosen to only acquire data for 300ms after an R-peak, although this is indicative and other values may be selected. The field of view is planned for each patient individually during adaptation of spatial resolution, slice number, and orientation. The shim settings are optimised accordingly, and the shim volumes are configured to substantially cover only the heart of each patient.

The data is acquired for one patient at a time for 300 ms after that patient's R-peak. Scanner settings are switched between patients dependent on which patient data has been acquired. Data acquisition progress is optionally balanced between patients to ensure that both measurements finish at substantially similar times. The acquired data is then sorted depending on which patient it originated from.

A further optional example provides grouping at least first and second patients using machine learning techniques applied to acquired measured data from previous experience. For example, out of a population of patients, a selection of a pair of patients is made based on individual parameters such as weight, height, and scan/rescan requirements with a name of optimal data acquisition. The proposed mechanism is not limited to identify similar patients for the scan. Optionally, a machine learning algorithm is applied to predict individual scan times for the patients based on their scan, personal data, and previous scans for further optimisation.

Fig. 2b illustrates an example of a display 82 (appropriately shielded and secured from magnetic effects) that may optionally be provided proximate to the bore of an during MRI scanner scanning. The display 82 is visible to a first 84 and a second 86 patient. The display 82 provides a visual stimulus, such as a video game the challenges the patients to adapt their breathing rhythm to a prescribed rhythm, for example. The prescribed rhythm is optionally not constant but has variations over time to keep the patient's attention. Additionally, the breathing rhythm of both patients is optionally measured using a vital signs camera, a breathing belt, or an MRI navigator and the patients are provided with a feedback of how well they are dear to the prescribed rhythm. Patients may thus be informed of how well other patients perform in a competitive gaming context with the intention of regularising or synchronising their breathing rate.

In the illustration of Fig. 2b, this game involves a graphic representation of a first pendulum 88 whose angular position varies according to breathing belt feedback from a first patient 84, and a second pendulum 90 whose angular position varies according to a breathing belt feedback from a second patient 86. Accordingly, the patients may be challenged to synchronise the angular positions of their pendula, and thereby their breathing phase and rate. Alternatively, the pendula can be operated without berating belt feedback to provide a visual indication of appropriate breathing phase to the patients.

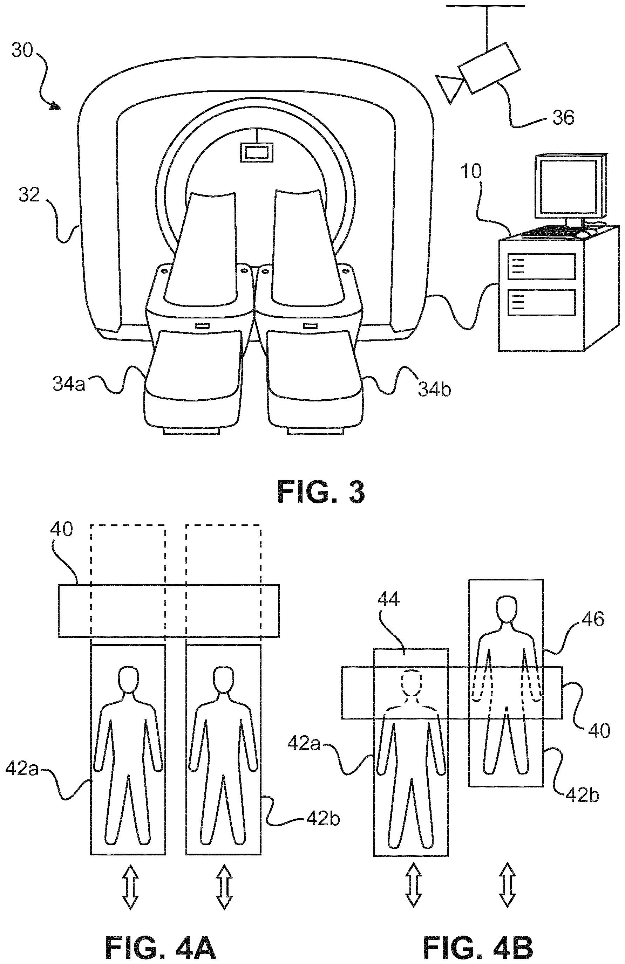

Fig. 3 schematically illustrates a general view of a medical imaging system 30 according to the third aspect capable of simultaneously and/or serially scanning two patients.

The medical imaging system 30 comprises:
- a medical scanner 32 configured to receive at least two patients comprising at least first 34a and second 34b movable patient supports, and
- an apparatus 10 according to the first aspect.

The first and second patient supports 34a, 34b of the medical imaging system are configured to receive first and second control signals from the output interface of the apparatus, and to position a first patient in a first imaging location of a medical scanner, and to position a second patient in a second imaging position of the medical scanner, wherein prior to, or during, the acquisition of first medical image data the medical scanner is configured using a first image configuration of the medical scanner, and wherein prior to, or during, the acquisition of second medical image data the medical scanner is configured using a second image configuration of the medical scanner.

The medical scanner 32 comprises a "wide bore" for accommodating at least two patients-typically a "wide bore" scanner is classified as having a diameter above 50 cm.

Optionally, the medical imaging system 30 further comprises a video camera having a field of view including the field of view of the medical scanner 32 configured to provide image information to the apparatus 10. Optionally, the medical imaging system 30 further comprises first and second physiological sensors (not shown) configured to obtain a first physiological trigger signal from the first patient, and a second physiological trigger signal from the second patient, and to communicate the first and/or second physiological trigger signals to the apparatus. For example, the first and second physiological signals may be cardiac signals, or breathing signals.

Fig. 4a and 4b schematically illustrate plan views of a simultaneous medical scanning process comprising two patients lying side-by-side.

Medical scanner 40 is therefore provided with a first patient support 42a and a second patient support 42b. In Fig. 4a), the patients are shown in a resting state before a scanning process begins. In Fig. 4b) the patients are shown during a process of medical scanning in which a first region 44 of the medical scanner accommodating the first patient is a first field of view, and a second region 46 accommodating the second patient is a second field of view.

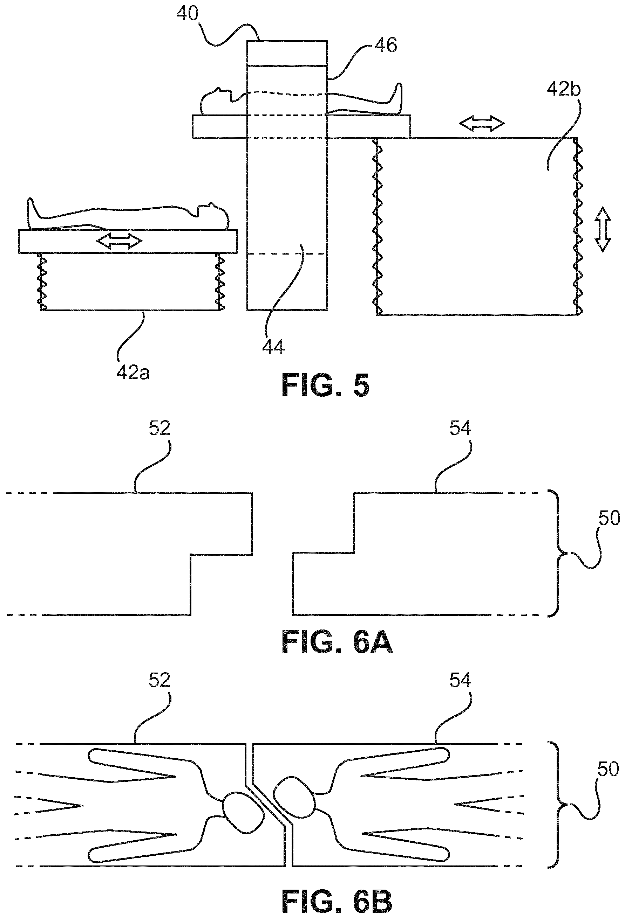

Fig. 5 schematically illustrates a side view of a simultaneous medical scanning process comprising two patients inside a medical scanner 40 in a stacked configuration. For example, the first patient support 42a is actually arranged through the aperture of the medical scanner 40 in alignment with the second patient support 42b. The second patient support 42b has a height-setting mechanism (not shown) capable of elevating the second patient to a second region of view 46 above the first region of view for accommodating the first patient.

Fig. 6a illustrates an interlocking first and second patient bed arrangement 50 in which the first patient bed 52 and the second patient bed 54 comprise interlocking mortise portions when the beds are brought together on the same horizontal level. Fig. 6b) illustrates a similar patient bed arrangement applied during a medical scan, in which the heads of the first and second patients have been redirected (angulated) to enable the first and second patient heads to be as near as possible to the iso centre of an MRI scanner.

Accordingly, both patient supports, in particular an upper one, can be lifted to different heights, optimally using bore size for different sized patients. At least one pair of rails is optionally provided at the side of the bore to support the patient supports mechanically when they are advanced into the bore.

Optionally, several pairs of rails at different heights may be provided so that supports can be parked at different heights, optimally using bore size for differently sized patients. This is advantageous, because especially in MRI, the image quality generally drops radially from the centre of the bore in all directions. In the case of MRI, the upper support may contain an array of receive coils 61 that can be used for simultaneous signal acquisition of both the lower and the upper patient.

Returning to the patient support design illustrated in Fig. 6b), optionally, up to 4 patient supports as illustrated in Fig. 6b) can be used with a MRI scanner. Scanning software can be used with the embodiment of the previous paragraph to enable scanning of two patients on each level positioned side-by-side. First and second patients may be inserted from a first side of the bore on the lower level, and two more patients may be inserted from the right hand side on the upper level.

Furthermore, the patients are now entered into the bore using up to four independent patient supports.

Optionally, head positions on the patient supports may be angulated and patient supports beveled to bring, for example, two heads as near as possible to the iso centre of an MRI scanner. Optionally, patient support may be equipped with headrests that enforce such positions and immobilize head position during scanning.

Optionally, patient supports may be equipped with two additional degrees of freedom to tilt them up or down and/or left or right such that they are not parallel to the principal axis (long axis) of the MRI scanner bore any more. This may be used for example to enable two heads to be positioned in the iso centre of the MRI scanner while the trunks of the patients are angulated to enable them to fit into the bore.

Optionally, the medical imaging system 30 further comprises a motion detection system configured to monitor motion of the first and/or second patients in the medical scanner, wherein the motion detection system is configured to predict a collision condition of the second patient with the first patient and/or the medical scanner based on a motion signal detected by the motion detection system, and to transmit the motion detection signal to the apparatus.

As noted above, patient throughput in diagnostic imaging is key and may be increased by simultaneous preparation scanning and removal of multiple patients in one scanner. Therefore, super-conducting wide-bore MR systems, "open MR" systems, and wide-bore CT systems are proposed that are equipped with multiple patient supports. According to this optional embodiment, a system for collision prevention is integrated based on depth cameras or laser systems. Optionally, existing patient data, image data, or newly acquired patient shape data is used to select and schedule suitable patients for simultaneous scanning, using conventional and machine learning approaches.

Optionally, a wide-bore MR or CT system may be equipped with multiple patient supports able to prepare, scan, and removed several patients simultaneously or alternately. As noted above, the upper and lower decks are preferentially designed to be advanced out of the bore to the two different ends of the bore.

The lower deck is designed conventionally, and the upper deck may be elevated (suspended by rails in the bore, or on an elavatable patient support, for example).

Optionally, In the case of MRI scanning, the upper deck may contain an array of RF receive coils 61 that may be used for simultaneous signal acquisition the both the lower and the other patient.

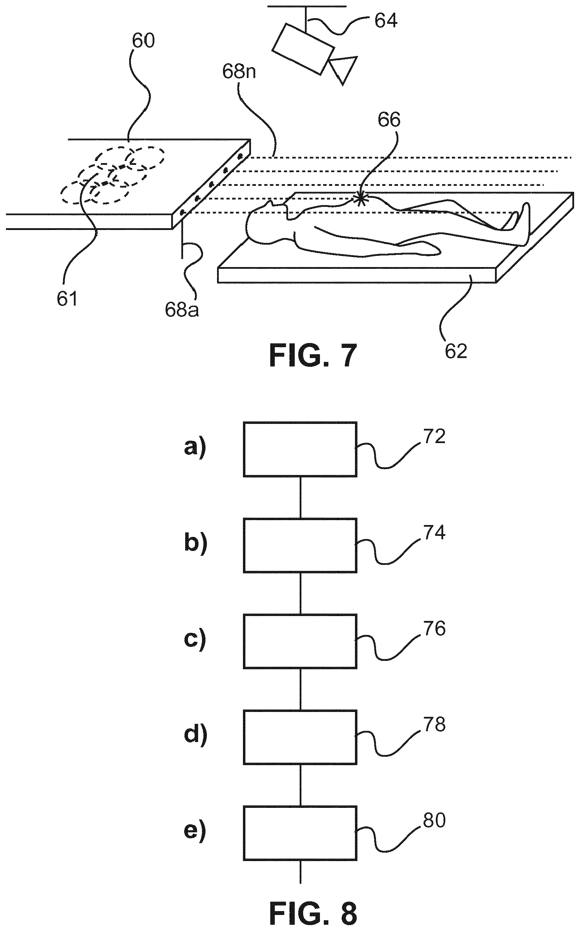

Fig. 7 schematically illustrates one example of a collision avoidance approach in a two-person stacked medical scanner. A first upper patient support 60 and a second patient support 62 are equipped with a system for collision prevention. Optionally, the system for collision prevention comprises a depth camera 64 mounted above the patient supports on one or both sides of the MRI scanner bore. Optionally, the depth camera is a Microsoft Kinect (TM) depth camera, or comparable type of depth camera. The cameras provide live depth images with an absolute depth accuracy of better than, for example, 2 mm and a lateral resolution of better than, for example, 4 mm. The depth camera images may be evaluated during positioning of the patients outside the bore. Optionally, the point of maximum elevation 66 of a patient is determined. In a first step, it is checked whether a patient will fit into the bought simultaneously with another patient. If not, an alarm may optionally be notified by the system enabling staff, or automatic handling hardware, to adjust the patient position or coil distribution or in the worst case even scan one patient alone. In the second step, optimal heights for both patient supports are calculated such that both patients are positioned as close as possible to the centre of the bore to ensure optimal image quality.

Optionally, a plurality of laser range meters 68a...68n are integrated into the lateral portion of the first patient support 60. Optionally, the laser range meters are positioned side-by-side at intervals of between 5 cm to 10 cm. Accordingly, a field of light rays parallel or substantially parallel to the length axis of the medical scanner is provided that can determine the distance to objects within a few metres with update rates of several Hz. As soon as the upper support is lifted to its operating height, both sets of range meters can determine whether any part of the upper patient will collide with the bore, and whether a part of the lower patient will collide with the upper support advancement the scanner.

Optionally the apparatus 10 according to the first aspect is provided, wherein the input interface 12 is configured to receive a motion signal from a motion detection system monitoring the medical scanner. The processor 14 is configured to monitor the second patient during their positioning at the second imaging location of the medical scanner using the motion signal, to predict a collision condition of the second patient with the first patient and/or the medical scanner based on the motion signal, and to halt the positioning of the second patient in the medical scanner, to adjust a positioning trajectory of the second patient based on the motion signal, and/or to change a second image acquisition configuration of the medical scanner based on the motion signal via a signal transmitted to the medical scanner via to output interface 16.

Optionally, the processor 14 is configured to acquire, via the input interface 12 physical measurement data of the first and the second patients, and the processor 14 is configured to signal to the medical scanner, via the output interface 16, to automatically adjust the spatial position in the medical scanner, or during positioning of the patients in the scanner, of a first patient support used to support the first patient and/or a second patient support used to support the second patient.

Optionally, the medical scanner of the embodiments discussed above is one of an MRI scanner, a CT scanner, a PET scanner, a SPECT scanner, an MRI-CT scanner, a PET-CT scanner.

Fig. 9 illustrates a method according to the second aspect.

Accordingly, a method for acquiring first medical image data from a first patient, and second medical image data from a second patient using the same medical scanner, comprising:
- positioning 72 a first patient in a first imaging location of a medical scanner;
- positioning 74 a second patient in a second imaging location of the medical scanner;
- acquiring 76 first medical image data of a first region of interest of the first patient using the medical scanner, wherein prior to, or during, the acquisition of the first medical image data the medical scanner is configured using a first image acquisition configuration of the medical scanner;
- acquiring 78 second medical image data of a second region of interest of the second patient using the medical scanner, wherein prior to, or during, the acquisition of the second medical image data the medical scanner is configured using a second image acquisition configuration of the medical scanner; and
- outputting 80 the first and second medical image data of the first and second patients.

Optionally, the method of the second aspect is provided wherein the first medical image data of the first region of interest of the first patient is acquired using a first scan protocol that is the same as a second scan protocol used to acquire the second medical image data of the second region of interest of the second patient.

Optionally, the method of the second aspect is provided wherein the first medical image data of the first region of interest of the first patient is acquired using a third scan protocol that is different to a fourth scan protocol used to acquire the second medical image data of the second region of interest of the second patient.

Optionally, the method of the second aspect further comprises
- obtaining a first physiological trigger signal of the first patient using a first physiological sensor monitoring the first patient, and acquiring the first medical image data at a time related to the first physiological trigger signal; and
- obtaining a second physiological trigger signal of the second patient using a second physiological sensor monitoring the second patient, and acquiring the second medical image data at a time related to the second physiological trigger signal.

According to a fourth aspect, there is provided computer program element for controlling an apparatus 10 according to the first aspect, and/or a medical imaging system 30 according to the third aspect, which, when the computer program element is executed by the apparatus and/or the medical imaging system, is adapted to perform the method of the third aspect.

According to a fifth aspect, there is provided a computer readable medium having stored the computer program element of the fourth aspect.

A computer program element might therefore be stored on a computer unit, which might also be part of an embodiment of the present invention. This computing unit may be adapted to perform or induce performance of the steps of the method described above.

Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both the computer program that has the invention installed from the beginning, and a computer program that by means of an update turns an existing program into a program that uses the invention. A computer program may be stored and/or distributed on a suitable medium, such as an optical storage media or a solid state medium supplied together with, or as a part of other hardware, but may also be distributed in other forms, such as via the Internet or other wired or wireless telecommunication systems.

However, the program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It should to be noted that embodiments of the invention are described with reference to different subject-matters. In particular, some embodiments are described with reference to method-type claims, whereas other embodiments are described with reference to the device-type claims. However, a person skilled in the art will gather from the above, and the following description, that unless otherwise notified, in addition to any combination of features belonging to one type of subject-matter, also any other combination between features relating to different subject-matters is considered to be disclosed with this application.

All features can be combined to provide a synergetic effect that is more than the simple summation of the features.

While the invention has been illustrated and described in detail in the drawings and foregoing description, such illustration and description are to be considered illustrative or exemplary, and not restrictive. The invention is not limited to the disclosed embodiments.

Other variations to the disclosed embodiments can be understood, and effected by those skilled in the art in practicing the claimed invention, from a study of the drawings, the disclosure, and the dependent claims.

In the claims, the word "comprising" does not exclude other elements or steps, and the indefinite article "a" or "an" does not exclude a plurality. A single processor, or other unit, may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. An apparatus (10) for acquiring first medical image data from a first patient, and second medical image data from a second patient using the same medical scanner, comprising:
- an input interface (12);
- a processor (14); and
- an output interface (16);
wherein the processor is configured to generate first control signals to position a first patient in a first imaging location of a medical scanner; and to communicate the first control signals to the medical scanner via the output interface (16);
wherein the processor (14) is configured to generate second control signals to position a second patient in a second imaging location of the medical scanner; and to communicate the second control signals to the medical scanner via the output interface;
wherein the processor (14) is configured to acquire first medical image data of a first region of interest of the first patient using the medical scanner via the input interface (12), wherein prior to, or during, the acquisition of the first medical image data the medical scanner is configured using a first image acquisition configuration generated by the processor (14) and transmitted to the medical scanner via the output interface (16);
wherein the processor (14) is configured to acquire second medical image data of a second region of interest of the second patient using the medical scanner via the input interface (12), wherein prior to, or during, the acquisition of the second medical image data the medical scanner is configured using a second image acquisition configuration of the medical scanner generated by the processor (14) and transmitted to the medical scanner via the output interface (16); and
wherein the output interface (16) is configured to output the first and second medical image data of the first and second patients.

2. The apparatus (10) according to claim 1,
wherein the first medical image data of the first region of interest of the first patient is acquired from the medical scanner via the input interface (12) using a first scan protocol that is the same as a second scan protocol used to acquire the second medical image data of the second region of interest of the second patient.

3. The apparatus (10) according to claim 1,
wherein the first medical image data of the first region of interest of the first patient is acquired from the medical scanner via the input interface (12) using a third scan protocol that is different to a fourth scan protocol used to acquire the second medical image data of the second region of interest of the second patient.

4. The apparatus (10) according to one of the preceding claims, wherein the processor (14) is further configured to:
- obtain, via the input interface (12) a first physiological trigger signal of the first patient using a first physiological sensor configured to monitor the first patient, and to acquire the first medical image data at a time related to the first physiological trigger signal; and
- obtain, via the input interface (12), a second physiological trigger signal of the second patient using a second physiological sensor configured to monitor the second patient, and to acquire the second medical image data at a time related to the second physiological trigger signal.

5. The apparatus (10) according to one of the preceding claims,
wherein the first medical image data is acquired from the medical scanner by the processor (14) simultaneously, or at an overlapping time period, relative to an acquisition time period of the second medical image data.

6. The apparatus (10) according to one of claims 1 to 4,
wherein the first medical image data is acquired from the medical scanner at a different time period compared to the acquisition time period of the second medical image data.

7. The apparatus (10) according to one of the preceding claims,
wherein the acquisition time of the first medical image data from the medical scanner occurs during the positioning of the second patient in the second imaging location of the medical scanner.

8. The apparatus (10) according to one of the preceding claims,
wherein the first and/or second image acquisition configuration generated by the processor (14) and transmitted to the medical scanner via the output interface (16) comprises a first field of view setting configured to obtain first medical image data of the first region of interest located on the first patient, and wherein the second image acquisition configuration generated by the processor (14) and transmitted to the medical scanner via the output interface (16) comprises a second field of view setting configured to obtain second medical image data of the second region of interest located on the second patient.

9. The apparatus (10) according to one of claims 1 to 6,
wherein the first and/or second image acquisition configurations of the medical scanner generated by the processor (14) and transmitted to the medical scanner via the output interface (16) comprise a field of view setting of the medical scanner that is the same for the acquisition of the first and the second medical image data.

10. The apparatus (10) according to one of the preceding claims,
wherein the first and second image acquisition configurations of the medical scanner generated by the processor (14) and transmitted to the medical scanner via the output interface (16) further comprise first and second shim settings, and the processor (14) is further configured to configure the medical scanner using the first shim setting prior to acquiring the first medical image data, and to configure the medical scanner using the second shim setting prior to acquiring the second medical image data.

11. The apparatus (10) according to one of the preceding claims,
wherein the input interface (12) is configured to receive a motion signal from a motion detection system monitoring the medical scanner; and
wherein the processor (14) is configured to monitor the second patient during their positioning at the second imaging location of the medical scanner using the motion signal, to predict a collision condition of the second patient with the first patient and/or the medical scanner based on the motion signal, and to halt the positioning of the second patient in the medical scanner, to adjust a positioning trajectory of the second patient based on the motion signal, and/or to change a second image acquisition configuration of the medical scanner based on the motion signal via a signal transmitted to the medical scanner via to output interface (16).

12. The apparatus (10) according claim 11,
wherein the processor (14) is configured to acquire, via the input interface (12) physical measurement data of the first and the second patients, and wherein the processor (14) is configured to signal to the medical scanner, via the output interface (16), to automatically adjust the spatial position in the medical scanner, or during positioning of the patients in the scanner, of a first patient support used to support the first patient and/or a second patient support used to support the second patient.

13. A method for acquiring first medical image data from a first patient, and second medical image data from a second patient using the same medical scanner, comprising:
- positioning (72) a first patient in a first imaging location of a medical scanner;
- positioning (74) a second patient in a second imaging location of the medical scanner;
- acquiring (76) first medical image data of a first region of interest of the first patient using the medical scanner, wherein prior to, or during, the acquisition of the first medical image data the medical scanner is configured using a first image acquisition configuration of the medical scanner;
- acquiring (78) second medical image data of a second region of interest of the second patient using the medical scanner, wherein prior to, or during, the acquisition of the second medical image data the medical scanner is configured using a second image acquisition configuration of the medical scanner; and
- outputting (80) the first and second medical image data of the first and second patients.

14. The method according to claim 13,
wherein the first medical image data of the first region of interest of the first patient is acquired using a first scan protocol that is the same as a second scan protocol used to acquire the second medical image data of the second region of interest of the second patient.

15. The method according to claim 13 or 14,
wherein the first medical image data of the first region of interest of the first patient is acquired using a third scan protocol that is different to a fourth scan protocol used to acquire the second medical image data of the second region of interest of the second patient.

16. The method according to one of claims 13 to 15, further comprising:
- obtaining a first physiological trigger signal of the first patient using a first physiological sensor monitoring the first patient, and acquiring the first medical image data at a time related to the first physiological trigger signal; and
- obtaining a second physiological trigger signal of the second patient using a second physiological sensor monitoring the second patient, and acquiring the second medical image data at a time related to the second physiological trigger signal.

17. A medical imaging system (30) comprising:
- a medical scanner (32) configured to receive at least two patients comprising at least first and second movable patient supports (34a, 34b); and
- an apparatus (10) according to one of claims 1 to 12;
wherein the first and second patient supports (34a, 34b) of the medical imaging system are configured to receive first and second control signals from the output interface of the apparatus, and to position a first patient in a first imaging location of a medical scanner, and to position a second patient in a second imaging position of the medical scanner, wherein prior to, or during, the acquisition of first medical image data the medical scanner is configured using a first image configuration of the medical scanner, and wherein prior to, or during, the acquisition of second medical image data the medical scanner is configured using a second image configuration of the medical scanner.

18. The medical imaging system according to claim 17, further comprising:
- first and second physiological sensors configured to obtain a first physiological trigger signal from the first patient, and a second physiological trigger signal from the second patient, and to communicate the first and/or second physiological trigger signals to the apparatus (10).

19. The medical imaging system (30) according to one of claim 17 or 18, further comprising:
- a motion detection system (36) configured to monitor motion of the first and/or second patients in the medical scanner (32), wherein the motion detection system is configured to predict a collision condition of the second patient with the first patient and/or the medical scanner based on a motion signal detected by the motion detection system (36), and to transmit the motion detection signal to the apparatus (36).
